Europäisches Patentamt

European Patent Office

Office européen des brevets

(11) Numéro de publication: **0 490 742 B1**

(12) **FASCICULE DE BREVET EUROPEEN**

(45) Date de publication de fascicule du brevet: **26.10.94** (51) Int. Cl.⁵: **C07C 1/20**, B01J 8/24

(21) Numéro de dépôt: **91403311.3**

(22) Date de dépôt: **06.12.91**

(54) **Procédé de régulation des conditions de fonctionnement d'un réacteur de conversion catalytique d'un composé oxygène utilisant une source de rayonnements ionisants.**

(30) Priorité: **10.12.90 FR 9015545**

(43) Date de publication de la demande:
**17.06.92 Bulletin 92/25**

(45) Mention de la délivrance du brevet:
**26.10.94 Bulletin 94/43**

(84) Etats contractants désignés:
**BE DE GB IT NL**

(56) Documents cités:
**EP-A- 0 308 027**

(73) Titulaire: **INSTITUT FRANCAIS DU PETROLE
4, Avenue de Bois Préau
F-92506 Rueil-Malmaison Cédex (FR)**

(72) Inventeur: **Hugues, François
10 Chemin du Clos Challans
F-69390 Charly Vernaison (FR)**
Inventeur: **Burzynski, Jean-Pierre
25, Rue du Brulet
F-69110 Sainte Foy Les Lyon (FR)**
Inventeur: **Vuillemot, Daniel
27, rue du l'Haye
F-69230 St. Genis Laval (FR)**
Inventeur: **Berreta, Alfred
Lotissement du Village
F-38200 De Vienne (FR)**
Inventeur: **Boute, Bernard
29, Domaine de la Côte
F-69530 Brignais (FR)**
Inventeur: **Pontier, Renaud
Le Village Luzinay
F-38200 Vienne (FR)**

Rank Xerox (UK) Business Services
(3.10/3.09/3.3.3)

## Description

L'invention concerne un procédé de conversion catalytique, en lit fluidisé entraîné, d'une charge comprenant au moins un composé oxygéné et plus spécialement au moins un alcool tel que le méthanol et/ou au moins un éther-oxyde tel que le diméthyléther, et l'utilisation d'un dispositif, pour la mise en oeuvre de ce procédé et pour la régulation des conditions de fonctionnement du réacteur, utilisant une source de rayonnements ionisants et, dans une forme particulière de réalisation, un appareil de mesure de la vitesse du solide catalytique au sein du réacteur.

Ce procédé s'applique plus particulièrement à la production d'hydrocarbures oléfiniques, notamment à usage pétrochimique, comprenant une majeure partie de composés ayant de 2 à 4 atomes de carbone dans leur molécule, en présence d'un catalyseur comme une zéolithe circulant dans un réacteur à lit fluidisé entraîné.

Une utilisation particulière donnée ici à titre d'exemple est la conversion catalytique du méthanol en hydrocarbures riches en éthylène et/ou en propylène.

Un des facteurs, essentiels à la production sélective d'oléfines légères, et plus particulièrement d'éthylène et/ou de propylène par conversion du méthanol, est la maîtrise du temps de contact entre la charge et le catalyseur. Ce contact entre la charge et le catalyseur doit être à la fois court et uniforme (ou régulier) dans le temps, ce qui impose une rapidité et une uniformité de la mise en contact entre les grains de catalyseur et la charge gazeuse ou liquide. Il est donc important de bien contrôler, en particulier, la quantité de charge introduite en liaison avec la quantité de catalyseur avec lequel elle entre en contact, ainsi que de garder constant le temps de contact entre la charge et le catalyseur de manière à obtenir la meilleure sélectivité possible en le ou les composés éthyléniques souhaités. En l'absence d'un tel contrôle, la tendance de la réaction est de conduire à une large gamme d'hydrocarbures saturés et insaturés allant généralement du méthane à des hydrocarbures ayant jusqu'à 10 atomes de carbone dans leur molécule ou plus, tels que ceux constituant habituellement la coupe essence.

L'association de conditions opératoires bien choisies et d'une telle régulation, en particulier du débit de solide dont la valeur peut notamment être obtenue par mesure de la densité du solide et avantageusement de la vitesse du solide dans le réacteur, permet en particulier de maintenir sensiblement constante au cours du temps la sélectivité en le ou les composés éthyléniques souhaités, ce qui n'était pas le cas dans les réalisations décrites dans l'art antérieur telles que par exemple celle décrite dans le brevet US-A-4046825.

Il est connu selon le brevet EP-A-308027 un procédé et un dispositif pour contrôler la masse volumique d'une suspension d'un échange de gaz et de particules solides, telles que du charbon transportée d'un récipient vers un moyen d'injection du mélange dans un réacteur, au moyen d'une source de rayonnement ionisant. Le signal obtenu est ensuite comparé avec un signal de référence et la masse volumique du mélange est ajustée en fonction du signal de référence de façon à optimiser les paramètres de la combustion du charbon dans le réacteur.

Le procédé de la présente invention permet de résoudre le problème d'une meilleure régulation du fonctionnement du réacteur de conversion catalytique, en particulier par une meilleure connaissance de la masse volumique du solide en mouvement, suivant au moins une direction ou en au moins une zone donnée, notamment en présence d'une charge vaporisée au contact du solide chaud, par exemple jusqu'à 700 °C. Il permet en outre de pallier, au moins en partie, les inconvénients liés à l'utilisation de sondes, et notamment de sondes de température, en contact avec le solide en mouvement, et en particulier des sondes de température qui sont normalement positionnées entre la zone d'introduction du solide catalytique et la zone d'introduction de la charge d'hydrocarbures ou à proximité de celle-ci. Il est cependant possible de conserver un certain nombre de sondes de température, par exemple celles situées dans la zone de désengagement du catalyseur ou dans la zone de régénération, ainsi que par exemples des sondes de pression ou d'autres sondes, par exemple des sondes optiques permettant la mesure de la vitesse du solide ces sondes fournissent des renseignements supplémentaires sur le fonctionnement du réacteur et peuvent permettre une optimisation plus poussée du fonctionnement dudit réacteur.

De façon plus précise, la présente invention concerne un procédé de conversion catalytique d'une charge, contenant une majeure partie d'au moins un composé oxygéné, en hydrocarbures oléfiniques riches en composés ayant de 2 à 4 atomes de carbone dans leur molécule, comprenant la conversion en lit fluidisé entraîné de ladite charge, dans une zone réactionnelle de conversion, dans des conditions appropriées, en présence d'un catalyseur sous forme de particules solides, ladite zone réactionnelle de forme allongée comprenant, à proximité d'une première extrémité, d'amont en aval dans le sens du déplacement de la charge, des moyens d'introduction d'au moins un fluide de fluidisation, des moyens d'introduction d'au moins un solide contenant des particules catalytiques et des moyens d'introduction de ladite charge, le procédé est caractérisé en ce qu'il comprend les étapes suivantes :

a) on émet un rayonnement ionisant par au moins une source de rayonnements ionisants et on détecte le rayonnement transmis par au moins un détecteur dudit rayonnement, ladite source et ledit détecteur étant positionnés à un niveau situé en aval, dans le sens du déplacement du solide, du niveau d'introduction du solide de manière à permettre la mesure de la masse volumique moyenne du solide en mouvement, selon au moins une direction déterminée sensiblement perpendiculaire à la direction de déplacement de la charge ou dans au moins une zone donnée, entre ladite source et ledit détecteur,

b) on mesure et on régule le débit de solide et/ou le débit de fluide de fluidisation et/ou le débit de la charge introduite dans la zone réactionnelle par des moyens de mesure et de régulation appropriés et

c) on ajuste, de façon continue ou périodique, au moins l'un des paramètres suivants de fonctionnement dudit procédé de conversion catalytique : la quantité de solide introduit dans la zone réactionnelle, le débit de fluide de fluidisation, et le débit de la charge introduite dans ladite zone réactionnelle par des moyens, par exemple de calculs programmables, permettant, à partir de l'acquisition de la valeur de la masse volumique, selon au moins une direction déterminée ou dans au moins une zone donnée et à un instant donné, d'effectuer la conversion de la charge dans des conditions stabilisées et préalablement déterminées, par comparaison avec les valeurs de référence des divers paramètres, valeurs par exemple mémorisées dans lesdits moyens de calculs programmables.

Dans une forme particulière de réalisation, la zone réactionnelle comprendra en outre au moins un moyen de détermination de la vitesse du solide en mouvement dans ladite zone réactionnelle.

Dans une autre forme de réalisation , elle pourra contenir éventuellement des moyens d'introduction d'un fluide d'entraînement, en amont de l'entrée de la charge dont le débit pourra être ajusté par les moyens de calculs programmables en liaison avec des moyens de mesure et de régulation de ce débit. Ce fluide d'entraînement favorise la fluidisation en phase diluée du solide.

L'utilisation d'une source de rayonnements ionisants et d'un détecteur dudit rayonnement positionnés dans le même plan, de part et d'autre de la zone réactionnelle, à un niveau choisi, permet, par la mesure de la variation de l'absorption de ce rayonnement (variation qui est directement liée à la variation de la masse volumique du solide en cet endroit de la mesure), de contrôler et par exemple de maintenir cette masse volumique entre deux valeurs extrêmes préalablement choisies. Il est possible d'effectuer une série de mesures suivant une série de directions dans le même plan et d'obtenir ainsi une cartographie de répartition de la masse volumique du solide dans le plan de mesure. Une telle cartographie peut être effectuée à l'aide d'un seul et même ensemble mobile, source et détecteur, ou à l'aide de plusieurs sources et de plusieurs détecteurs. Il est également possible d'effectuer plusieurs cartographies dans des plans situés à des niveaux différents, par exemple entre la zone d'introduction du solide et la zone d'introduction de la charge, ou en aval (dans le sens de déplacement du solide) de la zone d'introduction de la charge.

La source de rayonnements ionisants peut être positionnée à l'extérieur de la zone réactionnelle ou à l'intérieur de la zone réactionnelle, par exemple à proximité de sa périphérie. Il en est de même du détecteur de rayonnements. De manière préférée, la source et le détecteur sont placés à l'extérieur de la zone réactionnelle. Cette zone réactionnelle ou réacteur de forme allongée est habituellement un tube de section sensiblement circulaire et de diamètre sensiblement constant sur toute sa longueur, ayant un axe de symétrie.

Tout émetteur de rayonnements ionisants, ayant une puissance d'émission suffisante estimée en fonction des caractéristiques de l'appareillage utilisé, peut être employé. La source de rayonnements employée est habituellement une source de rayonnements alpha, béta, gamma, X ou une source de neutrons. On utilise le plus souvent une source de rayonnements gamma telle que par exemple une source scellée classique de Césium 137 ou une source de Cobalt 60. La puissance des sources utilisées est le plus souvent d'environ 3,7 à 370 x $10^{10}$ becquerels. Le détecteur du rayonnement est habituellement un détecteur classique sensible au rayonnements ionisants tel que par exemple un photomultiplicateur ou une chambre d'ionisation.

La ou les positions précises de la ou des sources de rayonnements ionisants et du ou des détecteurs correspondants, par exemple entre la zone d'introduction du solide comprenant des particules catalytiques et la zone d'introduction de la charge, ainsi que celle du ou des moyens de mesure de la vitesse du solide (lorsque ce ou ces moyens sont utilisés), par exemple en aval de la zone d'introduction de la charge dans le sens de circulation de cette charge, peut ou peuvent être aisément déterminées par l'homme du métier, en particulier en fonction de la taille de la zone de réaction et du débit moyen de solide choisi pour effectuer, dans les conditions employées, la conversion de la charge au sein du lit fluidisé entraîné, et également en fonction du ou des modes de régulations choisis.

Dans une première forme préférée de réalisation de l'invention, on ajuste de façon continue ou périodique le débit de fluide de fluidisation en liaison avec la mesure de la masse volumique et de préférence avec celle de la vitesse du solide effectuées à un niveau compris entre le niveau d'introduction du solide et le niveau d'introduction de la charge.

Dans une deuxième forme préférée de réalisation de l'invention, on ajuste de façon continue ou périodique le débit de charge en liaison avec la mesure de la masse volumique et de préférence avec celle de la vitesse du solide effectuées à un niveau situé en aval, dans le sens du déplacement du solide, du niveau d'introduction de la charge.

Dans une troisième forme préférée de réalisation de l'invention, on ajuste de façon continue ou périodique le débit de fluide d'entraînement en liaison avec la mesure de la masse volumique et de préférence avec celle de la vitesse du solide effectuées à un niveau compris entre le niveau d'introduction du solide et le niveau d'introduction de la charge.

Dans une quatrième forme préférée de réalisation de l'invention, on ajuste de façon continue ou périodique le débit de solide en liaison avec la mesure de la masse volumique et de préférence avec celle de la vitesse du solide effectuées à un niveau situé en aval, dans le sens du déplacement du solide, du niveau d'introduction de la charge.

Dans une cinquième forme de réalisation particulièrement performante, on combine les deux régulations décrites ci-avant en utilisant deux moyens de mesure dont l'un est situé entre le niveau d'introduction du solide et le niveau d'introduction de la charge et permet de réguler le débit de fluide d'entraînement et dont l'autre est situé en aval du niveau d'introduction de la charge et permet de réguler le débit de solide et de préférence on utilise également au moins un moyen de mesure de la vitesse du solide.

Enfin dans une autre forme de réalisation de l'invention, on ajuste de façon continue ou périodique le débit de solide introduit dans la zone de réaction, le débit de fluide d'entraînement et le débit de fluide de fluidisation.

La puissance de la source est elle-même choisie, en particulier en fonction des deux premiers paramètres cités ci-avant, de manière à pouvoir permettre une mesure de la masse volumique dans de bonnes conditions de sensibilité. Dans une forme préférée de mise en oeuvre de la présente invention, les moyens de mesure de la masse volumique, c'est-à-dire la source de rayonnements ionisants et le détecteur qui lui est associé, sont situés à un niveau suffisamment éloigné de la zone d'introduction du solide de manière à ce que la mesure de la masse volumique ne soit pas effectuée au niveau de la zone d'agitation importante qui existe à proximité immédiate de cette zone d'introduction du solide, et de même, pour des raisons similaires, les moyens de mesures seront situés de préférence à un niveau suffisamment éloigné de la zone d'introduction de la charge, et avantageusement en aval de celle-ci.

Les signaux obtenus à la sortie du détecteur et éventuellement ceux résultant de la mesure de la vitesse du solide sont habituellement envoyés, éventuellement après transformation ou traitement par exemple à l'aide d'un analyseur de signaux tel qu'un analyseur multicanaux, aux moyens de calculs programmables. Ces signaux permettent après étalonnage de connaître la masse volumique du solide dans la partie traversée par le rayonnement ionisant. C'est habituellement cette valeur calculée de la masse volumique qui est envoyée aux moyens de calculs programmables. Ces moyens de calculs programmables renferment habituellement un ordinateur comportant un programme adapté à l'analyse comparative de diverses valeurs par rapport à des valeurs seuils. Ces valeurs sont ainsi le plus souvent traitées suivant un modèle mathématique d'écoulement des solides dans une enceinte de forme allongée. A titre d'exemple non limitatif de modèle mathématique pouvant être utilisé pour déterminer le débit de solide, la demanderesse a utilisé le modèle suivant :

$$DC = \frac{3\,(M - a)}{\left(R^2 + 2y^2\right)\left(Ae^{-z} + B\right)}$$

Dans ce modèle, DC représente le débit (en tonne par heure) du catalyseur, M représente la valeur de la masse volumique (en kilogramme par mètre cube) mesurée à partir du ou des signaux obtenus à la sortie des moyens de mesures, R représente le rayon moyen (en mètre) de la zone réactionnelle de forme allongée dans le plan où l'on effectue la mesure, et A,B et a représentent trois constantes déterminées par étalonnage, dont les valeurs sont en particulier liées à la position de la source par rapport à la zone réactionnelle, à la puissance de la source et aux dimensions de la zone réactionnelle, e est la représentation de la fonction exponentielle, z est un nombre (exprimé en mètre) égal à la distance entre la zone de mesure et la zone d'entrée du catalyseur et y est la distance, en mètre, entre le centre de l'enceinte de forme allongée et la direction suivant laquelle est effectuée la mesure.

Selon une autre forme de réalisation de l'invention, le débit de solide peut aussi être déterminé en utilisant le ou les moyens de mesure de la vitesse du solide. Par corrélation de la mesure de la masse volumique avec la mesure de la vitesse du solide dans la zone réactionnelle, il sera donc possible de connaître et de contrôler et par exemple de maintenir le débit de solide dans ladite zone réactionnelle entre deux valeurs extrêmes préalablement choisies.

Le ou les moyens de détermination de la vitesse du solide dans la zone réactionnelle peut être n'importe quel moyen bien connu de l'homme du métier permettant cette mesure. A titre d'exemple d'un tel moyen, on peut citer l'utilisation de la vélocimétrie Doppler à l'aide d'une sonde optique et d'un faisceau laser.

Les moyens de calculs programmables comparent la ou les valeurs fournies à la ou aux valeurs de référence choisies et lorsqu'il y a déviation par rapport à cette ou à l'une de ces valeurs de référence choisies, ces moyens modifient ou ajustent au moins l'un des paramètres suivants du fonctionnement du procédé de conversion catalytique : la quantité de solide introduit, le débit de fluide de fluidisation, le débit de fluide d'entraînement si nécessaire et le débit de la charge introduite dans ladite zone réactionnelle, cette modification étant effectuée de manière à permettre un retour aussi rapide que possible aux conditions de fonctionnement préalablement choisies par l'homme du métier. Cette modification peut être effectuée de façon continue ou périodique, simultanément ou de façon décalée par rapport à l'instant de la ou des mesures de la masse volumique et de celle(s) de la vitesse du solide lorsque cette ou ces mesures sont effectuées.

La modification des débits du solide ou des divers fluides est habituellement effectuée par action sur des vannes automatiques, commandées par les moyens de calculs programmables, installées sur les divers conduits d'introduction de ce solide ou de ces fluides dans la zone de réaction.

Dans une forme particulièrement performante de mise en oeuvre du procédé de conversion catalytique de la présente invention, la zone réactionnelle comprend au moins deux points d'introduction de la charge et de préférence une pluralité de points d'introduction de ladite charge. La masse volumique du solide est alors mesurée ou évaluée à proximité de chacun de ces points d'introduction et la quantité de charge introduite en chaque point est régulée au cours du temps en relation avec la masse volumique de solide mesurée à proximité dudit point d'introduction de la charge.

Dans les figures annexées, illustrant un mode particulier de réalisation de l'invention, les organes similaires sont désignés par les mêmes chiffres ou lettres de référence.

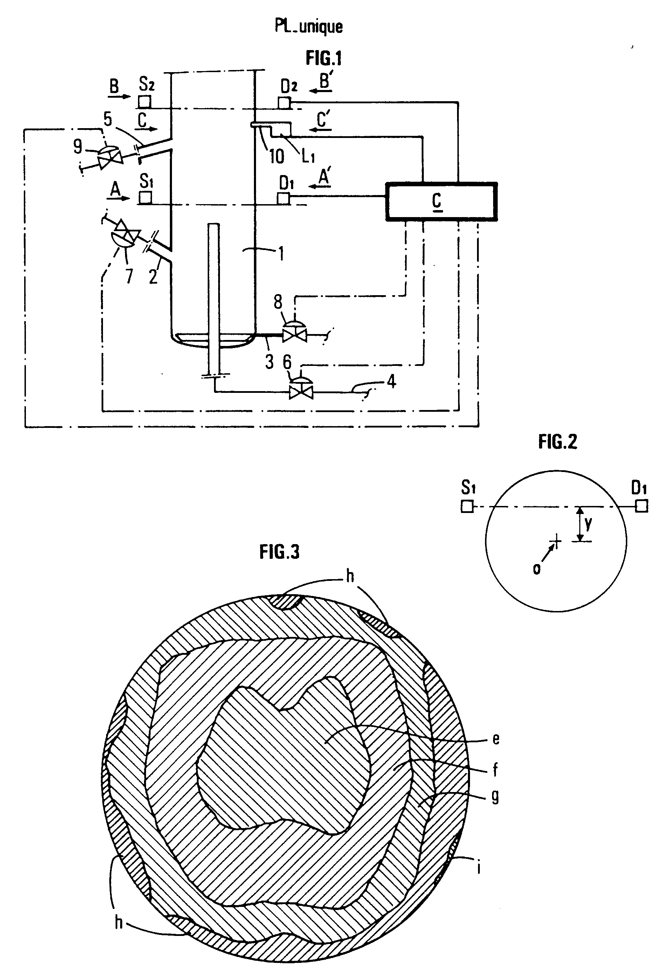

La figure 1 représente, selon une forme de réalisation préférentielle, le schéma de principe de la boucle de régulation. Les organes de régulation du débit du gaz d'entraînement, du solide catalytique, du gaz de fluidisation et de la charge, sont schématisés par les vannes (6, 7, 8 et 9). Le catalyseur frais et/ou régénéré arrive, à proximité d'une extrémité du réacteur (1), par le conduit (2) comportant une vanne réglable (7). Par le conduit (3) comportant une vanne réglable (8), on introduit le fluide de fluidisation en amont de l'arrivée du catalyseur dans le réacteur. Par le conduit (4), comportant une vanne réglable (6), débouchant dans le réacteur (1) à un niveau situé entre la zone d'introduction du catalyseur et la zone d'introduction de la charge, on introduit le fluide d'entraînement. La charge est introduite par le conduit (5) comportant une vanne réglable (9). Elle est vaporisée en présence du catalyseur. La mesure de la masse volumique est effectuée suivant les axes AA' et BB' aux niveaux desquels sont positionnés les sources S1 et S2 de rayonnements ionisants et les détecteurs D1 et D2 du rayonnement ionisant émis par les sources et ayant traversé le solide en mouvement dans le réacteur (1). La mesure de la vitesse est effectuée à l'aide du laser L1 et de la sonde optique (10) suivant l'axe CC'. Les détecteurs D1 et D2 et le système de mesure de la vitesse (comprenant la sonde optique (10) et le laser L1) sont reliés aux moyens de calcul programmables C qui sont eux mêmes reliés aux vannes (6), (7), (8) et (9) dont ils commandent l'ouverture.

La figure 2 représente une coupe du réacteur suivant l'axe AA' et montre la direction selon laquelle la mesure du rayonnement transmis à travers le solide en mouvement est effectuée. La distance de la corde selon laquelle la mesure est effectuée au centre "o" du réacteur (1) est égale à y. Suite à de très nombreux essais, la demanderesse a déterminé qu'il existe une corde selon laquelle la valeur mesurée de la masse volumique du solide en mouvement est pratiquement égale à la valeur moyenne de cette masse volumique, calculée sur l'ensemble du plan de la section. Dans la majorité des cas, il est donc possible de n'effectuer la mesure de la masse volumique que suivant cette corde tout en conservant une très bonne possibilité de régulation des conditions de fonctionnement du réacteur.

La figure 3 représente un gammagramme obtenu par une série de mesures effectuées dans un plan sensiblement perpendiculaire à l'axe du réacteur et passant par l'axe AA'. Les zones e, f, g, h et i sont des zones dans lesquelles la masse volumique est comprise entre deux valeurs extrêmes (zones d'isodensités).

Le tableau 1 représente un exemple d'algorithme suivi pour réguler les conditions de fonctionnement d'un réacteur de conversion catalytique.

La présente invention concerne également un dispositif pour réguler les conditions de fonctionnement d'un réacteur de conversion catalytique en lit fluidisé entraîné, d'une charge contenant une majeure partie d'au moins un composé oxygéné, en hydrocarbures oléfiniques riches en composés ayant de 2 à 4 atomes de carbone dans leur molécule, de forme allongée, comprenant, à proximité d'une première extrémité, d'amont en aval dans le sens du déplacement de la charge, des moyens d'introduction d'au moins un fluide de fluidisation, des moyens d'introduction d'au moins un solide contenant des particules catalytiques, des moyens d'introduction d'au moins un fluide d'entraînement si nécessaire et des moyens d'introduction de ladite charge caractérisé en ce que ledit dispositif comprend :

a) au moins une source de rayonnements ionisants et au moins un détecteur dudit rayonnement, ladite source et ledit détecteur étant positionnés à un niveau situé en aval, dans le sens du déplacement du solide, du niveau d'introduction du solide de manière à permettre la mesure de la masse volumique moyenne du solide en mouvement, selon au moins une direction déterminée sensiblement perpendiculaire à la direction de déplacement de la charge ou dans au moins une zone donnée, entre ladite source et ledit détecteur, ce dernier étant relié à des moyens de calcul programmables définis ci-dessous,

b) des moyens de mesure et de régulation de la quantité de solide et/ou du débit de fluide de fluidisation et/ou du débit de fluide d'entraînement et/ou du débit de la charge introduits dans le réacteur et

c) des moyens, par exemple de calculs programmables connectés aux moyens de mesure et de régulation et au dit détecteur, permettant, à partir de l'acquisition de la valeur de la masse volumique, selon au moins une direction déterminée ou dans au moins une zone donnée et à un instant donné, d'effectuer la conversion de la charge dans des conditions stabilisées et préalablement déterminées, par comparaison avec les valeurs de référence des divers paramètres, valeurs par exemple mémorisées dans lesdits moyen de calcul programmables, en ajustant, de façon continue ou périodique, au moins l'un des paramètres suivants de fonctionnement dudit procédé de conversion catalytique : la quantité de solide introduit, le débit de fluide de fluidisation, le débit de fluide d'entraînement si nécessaire et le débit de la charge introduite dans ladite zone réactionnelle.

Dans une forme préférée de réalisation, le dispositif de l'invention comprend en outre au moins un moyen de détermination de la vitesse du solide en mouvement dans ladite zone réactionnelle.

Dans le cadre de la présente invention, il n'est pas fait menton des solides et catalyseurs employés, ni des divers fluides utilisés qui sont des données classiques bien connues de l'homme de la conversion catalytique des composés oxygénés en hydrocarbures oléfiniques. Le fluide d'entraînement est le plus souvent formé par une fraction du fluide de fluidisation.

On utilise de préférence des catalyseurs zéolithiques et de la vapeur d'eau, au moins un gaz inerte ou des hydrocarbures gazeux comme fluide de fluidisation.

Les conditions opératoires relatives aux procédés de conversion de charges oxygénées comme le méthanol sont par exemples décrites dans les brevets suivants : US-A-4689205, US-A-3969426 et US-A-4229608, mais aucun de ces brevets ne décrit le contrôle des conditions de marche du réacteur selon la présente invention.

Les conditions opératoires de la conversion de la charge oxygénée sont en général les suivantes :
- la charge, qui le plus souvent contient un alcool et habituellement le méthanol, renferme généralement de 50 à 100 %, de préférence de 70 à 90 % en poids de composés oxygénés et de 0 à 50 %, de préférence de 5 à 30 % en poids d'eau. La charge peut également contenir un gaz inerte tel que par exemple l'azote seul ou en mélange avec la vapeur d'eau dans la proportion indiquée ci-avant pour la vapeur d'eau. Dans le cas le plus fréquent où la charge contient du méthanol, elle peut être constituée par du méthanol brut tel que par exemple du méthanol provenant d'une unité de synthèse de ce composé à partir d'un gaz contenant du monoxyde de carbone,
- la température d'injection est habituellement égale à la température de rosée de la charge,
- la pression absolue régnant dans l'unité de conversion est habituellement de 0,1 à 0,5 mégapascal (MPa) et le plus souvent d'environ 0,2 MPa,
- la température de la zone réactionnelle est habituellement de 500 à 620 °C et le plus souvent de 550 à 590 °C,
- le temps de séjour dans la zone réactionnelle est habituellement de 0,05 à 10 secondes et le plus souvent de 0,5 à 3 secondes,
- la vitesse des gaz dans la zone réactionnelle est habituellement de 0,5 à 40 m/s et le plus souvent de 1 à 20 m/s,
- la vitesse des solides dans la zone réactionnelle est habituellement voisine de celle des gaz et généralement de 0,5 à 40 m/s et le plus souvent de 1 à 20 m/s.

6

EP 0 490 742 B1

Les conditions opératoires sont choisies pour que la composition des produits obtenus exprimée par le rendement en carbone en composés éthyléniques ayant de 2 à 4 atomes de carbone dans leur molécule soit supérieure à 70 % et de préférence supérieure à 80 %. Les produits obtenus peuvent être particulièrement riches en éthylène (rendement en carbone pour l'éthylène supérieur à 40 %) ou particulièrement riches en propylène (rendement en carbone pour le propylène supérieur à 60 %).

La masse volumique des solides est habituellement de 10 à 700 kilogrammes par mètre cube (kg/m3) et le plus souvent de 20 à 400 kg/m3.

Le rapport C/O du débit massique de catalyseur au débit massique de charge est habituellement d'environ 2 : 1 à environ 50 : 1 et le plus souvent d'environ 5 : 1 à environ 30 : 1.

```
                    ┌──────────────────────────────────┐
                    │   Initialisation des paramètres   │
                    └──────────────────────────────────┘
                                     │
                                     ▼
             ┌────────────────────────────────────────────────┐
             │  Acquisition des valeurs venant des détecteurs  │
             └────────────────────────────────────────────────┘
                                     │
                                     ▼
                      ┌──────────────────────────┐
                      │  Traitement des valeurs   │
                      └──────────────────────────┘
                                     │
                                     ▼
            ┌──────────────────────────────────────────────────┐
            │  Affichage des valeurs   Sortie vers enregistreur │
            └──────────────────────────────────────────────────┘
                                     │
                                     ▼
           ┌────────────────────────────────────────────────────┐
           │ Comparaison des valeurs aux valeurs de consigne     │
           └────────────────────────────────────────────────────┘
                    │                              │
                    ▼                              ▼
         ┌───────────────────────┐      ┌───────────────────────┐
         │  Niveaux valeurs bon   │      │  Valeurs hors consignes │
         └───────────────────────┘      └───────────────────────┘
                    │                              │
                    ▼                              ▼
             ┌──────────────┐             ┌──────────────┐
             │  Pas d'action │             │  Action O/F   │
             └──────────────┘             └──────────────┘

   ┌──────────────┐   ┌──────────────┐   ┌──────────────┐   ┌──────────────┐
   │ Vanne débit  │   │ Vanne débit  │   │ Vanne débit  │   │ Vanne débit  │
   │    charge    │   │     gaz      │   │    solide    │   │    fluide    │
   │              │   │ entrainement │   │              │   │ fluidization │
   └──────────────┘   └──────────────┘   └──────────────┘   └──────────────┘
```

## TABLEAU I

7

**Revendications**

1. Procédé de conversion catalytique d'une charge contenant une majeure partie d'au moins un composé oxygéné, en hydrocarbures oléfiniques riches en composés ayant de 2 à 4 atomes de carbone dans leur molécule, comprenant la conversion en lit fluidisé entraîné de ladite charge, dans une zone réactionnelle de conversion, dans des conditions appropriées, en présence d'un catalyseur sous forme de particules solides, ladite zone réactionnelle de forme allongée comprenant, à proximité d'une première extrémité, d'amont en aval dans le sens du déplacement de la charge, des moyens d'introduction d'au moins un fluide de fluidisation, des moyens d'introduction d'au moins un solide contenant des particules catalytiques, et des moyens d'introduction de ladite charge, le procédé comportant l'émission d'un rayonnement ionisant et la détection d'un rayonnement transmis, le procédé étant caractérisé en ce qu'il comprend les étapes suivantes:

   a) on émet le rayonnement ionisant par au moins une source de rayonnements ionisants et on détecte le rayonnement transmis par au moins un détecteur dudit rayonnement, ladite source et ledit détecteur étant positionnés à un niveau situé en aval, dans le sens du déplacement du solide, du niveau d'introduction du solide de manière à permettre la mesure de la masse volumique moyenne du solide en mouvement, selon au moins une direction déterminée sensiblement perpendiculaire à la direction de déplacement de la charge ou dans au moins une zone donnée, entre ladite source et ledit détecteur,

   b) on mesure et on régule le débit de solide et/ou le débit de fluide de fluidisation et/ou le débit de la charge introduite dans la zone réactionnelle par des moyens de mesure et de régulation appropriés et

   c) on ajuste, de façon continue ou périodique, au moins l'un des paramètres suivants de fonctionnement dudit procédé de conversion catalytique : la quantité de solide introduit dans la zone réactionnelle, le débit de fluide de fluidisation, et le débit de la charge introduite dans ladite zone réactionnelle par des moyens, par exemple de calculs programmables, permettant, à partir de l'acquisition de la valeur de la masse volumique, selon au moins une direction déterminée ou dans au moins une zone donnée et à un instant donné, d'effectuer la conversion de la charge dans des conditions stabilisées et préalablement déterminées, par comparaison avec les valeurs de référence des divers paramètres, valeurs par exemple mémorisées dans lesdits moyens de calculs programmables.

2. Procédé selon la revendication 1 dans lequel lors de l'étape a, on détermine en outre la vitesse du solide en mouvement par au moins un moyen de détermination de la vitesse du dit solide.

3. Procédé selon la revendication 1 ou 2 dans lequel la charge est introduite à l'état gazeux.

4. Procédé selon l'une des revendications 1 à 3 dans lequel la charge comprend au moins un alcool, de préférence le méthanol.

5. Procédé selon l'une des revendications 1 à 4 dans lequel la charge contient de l'eau.

6. Procédé selon l'une des revendications 1 à 5 dans lequel on effectue une série de mesures de la masse volumique moyenne du solide, dans un plan donné, suivant une série de directions et de préférence suivant une corde située à une distance appropriée du centre de la zone réactionnelle.

7. Procédé selon l'une des revendications 1 à 6 dans lequel la source de rayonnements et le détecteur dudit rayonnement sont situés à l'extérieur de la zone réactionnelle.

8. Procédé selon l'une des revendications 1 à 7 dans lequel on ajuste de façon continue ou périodique le débit de fluide de fluidisation en liaison avec la mesure de la masse volumique du solide.

9. Procédé selon l'une des revendications 1 à 7 dans lequel on ajuste de façon continue ou périodique le débit de charge, en liaison avec la mesure de la masse volumique du solide.

10. Procédé selon l'une des revendications 1 à 7 dans lequel on ajuste de façon continue ou périodique la quantité de solide introduite dans la zone de réaction, en liaison avec la mesure de la masse volumique du solide.

**11.** Procédé selon l'une des revendications 1 à 7 dans lequel on introduit un fluide d'entraînement en amont de l'entrée de la charge et on ajuste de façon continue ou périodique son débit par les moyens de calcul, en liaison avec la mesure de la masse volumique du solide.

**12.** Procédé selon l'une des revendications 1 à 7 et 11 dans lequel on ajuste simultanément de façon continue ou périodique la quantité de solide introduite dans la zone de réaction et le débit de fluide d'entraînement en liaison avec la mesure de la masse volumique du solide et de préférence avec celle de sa vitesse.

**13.** Procédé selon l'une des revendications 1 à 12 dans lequel la zone réactionnelle comprend une pluralité de points d'introduction de la charge à travers lesquels on introduit une quantité de charge en relation avec la masse volumique de solide mesurée à proximité dudit point d'introduction de la charge et on régule cette quantité de charge en fonction de la masse volumique mesurée.

**Claims**

**1.** Catalytic conversion process of a charge containing a majority of at least one oxygen compound, with olefinic hydrocarbons rich in compounds having molecules containing 2 to 4 carbons atoms, comprising the conversion of said charge in an entrained fluidised bed, in a conversion reaction zone, under suitable conditions, in the presence of a catalyst in the form of solid particles, said reaction zone having an elongated shape comprising, in the proximity of a first extremity, from upstream to downstream in the direction of displacement of the charge, means for introduction of at least one fluidisation fluid, means for introduction of at least one solid containing catalytic particles, and means for introduction of said charge, the process including the emission of ionising radiation and the sensing of radiation transmitted, the process being characterised in that it includes the following steps:

a) the ionising radiation is emitted by at least one ionising radiation source and the radiation transmitted is sensed by at least one sensor of said radiation, said source and said sensor being positioned at a level located downstream in the direction of displacement of the solids, at the level of introduction of the solids, in such a manner as to permit measurement of the average density of the solids in movement, according to at least one predetermined direction substantially perpendicular to the direction of displacement of the charge or in at least one given zone between said source and said detector,

b) the discharge rate of solid and/or the discharge rate of fluidisation fluid and/or the discharge rate of the charge introduced into the reaction zone is measured and controlled by appropriate measuring and control means, and

c) at least one of the following function parameters of said catalytic conversion process is continuously or periodically adjusted: the amount of solids introduced into the reaction zone, the discharge rate of the fluidisation fluid, and the discharge rate of the charge introduced into said reaction zone by, for example, programmable calculation means, allowing, following acquisition of the value of the density, according to at least one predetermined direction or in at least one given zone, and at a given moment, for conversion of the charge to be carried out under stabilised and previously determined conditions, by comparison with reference values for the various parameters, for example, values memorised in said programmable calculation means.

**2.** Process according to claim 1 in which moreover during step a, the speed of the solids in movement is determined by at least one means for determination of the speed of said solids.

**3.** Process according to claim 1 or 2, in which the charge is introduced in a gaseous state.

**4.** Process according to one of claims 1 to 3, in which the charge contains at least one alcohol, preferably methanol.

**5.** Process according to one of claims 1 to 4, in which the charge contains water.

**6.** Process according to one of claims 1 to 5, in which a series of measurements of the average density of the solids is carried out on a given plane, following a series of directions and preferably following a chord located at a suitable distance from the centre of the reaction zone.

7. Process according to one of claims 1 to 6, in which the radiation source and the sensor of said radiation are located outside the reaction zone.

8. Process according to one of claims 1 to 7, in which the discharge rate of the fluidisation fluid is continuously or periodically adjusted in connection with the measurement of the density of the solids.

9. Process according to one of claims 1 to 7, in which the discharge rate of the charge is continuously or periodically adjusted in connection with the measurement of the density of the solids.

10. Process according to one of claims 1 to 7, in which the quantity of solids introduced into the reaction zone is adjusted continuously or periodically in connection with the measurement of the density of the solids.

11. Process according to one of claims 1 to 7, in which an entrainment fluid is introduced upstream of the entrance of the charge and the discharge rate thereof is adjusted continuously or periodically by calculation means, in connection with the measurement of the density of the solids.

12. Process according to one of claims 1 to 7 and 11 in which the quantity of solids introduced into the reaction zone and the discharge rate of the entrainment fluid are adjusted simultaneously in a continuous or periodic manner in connection with the measurement of the density of the solids and preferably with that of its speed.

13. Process according to one of claims 1 to 12 in which the reaction zone comprises a plurality of introduction points for the charge through which a quantity of charge is introduced in relation to the density of solids measured in the proximity of said charge introduction point, and this quantity of charge is controlled according to the density measured.

**Patentansprüche**

1. Verfahren zur katalytischen Umwandlung einer einen größeren Teil wenigstens einer sauerstoffhaltigen Verbindung enthaltenden Charge in olefinische Kohlenwasserstoffe, die reich an Verbindungen, welche 2 bis 4 Kohlenstoffatome in ihrem Molekül haben, sind, umfassend die Umwandlung dieser Charge im Wirbelschleppbett in einer Umwandlungsreaktionszone unter geeigneten Bedingungen, in Anwesenheit eines Katalystors in Form von Feststoffpartikeln umfassend, wobei diese Reaktionszone länglicher Gestalt benachbart einem ersten Ende Von der Anströmseite zur Abströmseite in Bewegungsrichtung der Charge gesehen, Einführungsmittel wenigstens eines Fluidisierungsfluids, Mittel zum Einführen wenigstens eines katalytische Partikel enthaltenden Feststoffs und Mittel zum Einführen dieser Charge umfaßt, und das Verfahren die Aussendung einer ionisierenden Strahlung und das Erfassen einer übertragenen Strahlung umfaßt, wobei das Verfahren dadurch gekennzeichnet ist, daß es die folgenden Stufen umfaßt:

a) man sendet die ionisierende Strahlung über wenigstens eine Quelle für ionisierende Strahlungen aus und erfaßt die übertragene Strahlung vermittels wenigstens eines Detektors dieser Strahlung, wobei diese Quelle und dieser Detektor auf einem Niveau positioniert sind, das in Bewegungsrichtung des Feststoffs abströmseitig des Einführungsniveaus des Feststoffs derart vorgesehen ist, daß die Messung der mittleren volumenbezogenen Masse des in Bewegung befindlichen Feststoffs entsprechend wenigstens einer Richtung möglich wird, die im wesentlichen senkrecht zur Bewegungsrichtung der Charge oder in wenigstens einer gegebenen Zone zwischen dieser Quelle und diesem Detektor bestimmt wird,

b) man mißt und man stellt ein den Feststoffdurchsatz und/oder den Durchsatz an Fluidisierungsfluid und/oder den Durchsatz der Charge, die in die Reaktionszone über geeignete Meß- und Einstellmittel eingeführt wird und

c) man stellt in kontinuierlicher Weise oder periodisch einen der folgenden Arbeitsparameter dieses katalytischen Umwandlungsverfahrens ein: die Menge des in die Reaktionszone eingeführten Feststoffs, den Durchsatz an Fluidisierungsfluid und den Durchsatz der in dieser Reaktionszone eingeführten Charge über Mittel, beispielsweise zur programmierbaren Berechnung, die es ermöglichen, ausgehend vom Erfassen des Werts der volumenbezogenen Masse gemäß wenigstens einer bestimmten Richtung oder in wenigstens einer gegebenen Zone und zu einem gegebenen Augenblick die Umwandlung der Charge unter stabilisierten und vorher festgelegten Bedingungen durch Vergleich mit den Bezugswerten der verschiedenen Parameter, beispielsweise Werten, die in diesen programmierbaren Rechnermitteln gespeichert sind, auszuführen.

2. Verfahren nach Anspruch 1, bei dem man während der Stufe a zusätzlich die Geschwindigkeit des in Bewegung befindlichen Feststoffs über wenigstens ein Mittel zur Bestimmung dieses Feststoffs bestimmt.

3. Verfahren nach Anspruch 1 oder 2, bei dem die Charge im gasförmigen Zustand eingeführt wird.

4. Verfahren nach einem der Ansprüche 1 bis 3, bei dem die Charge wenigstens einen Alkohol, vorzugsweise Methanol, umfaßt.

5. Verfahren nach einem der Ansprüche 1 bis 4, bei dem die Charge Wasser enthält.

6. Verfahren nach einem der Ansprüche 1 bis 5, bei dem man eine Reihe von Messungen der mittleren volumenbezogenen Masse des Feststoffs in einer gegebenen Ebene entsprechend einer Reihe von Richtungen und vorzugsweise längs einer Sehne durchführt, die sich unter einer geeigneten Entfernung von der Mitte der Reaktionszone befindet.

7. Verfahren nach einem der Ansprüche 1 bis 6, bei dem sich die Strahlungsquelle und der Detektor für die Strahlung außerhalb der Reaktionszone befinden.

8. Verfahren nach einem der Ansprüche 1 bis 7, bei dem man in kontinuierlicher oder periodischer Weise den Durchsatz des Fluidisierungsfluids in Verbindung mit der Messung der volumenbezogenen Masse des Feststoffs einstellt.

9. Verfahren nach einem der Ansprüche 1 bis 7, bei dem man in kontinuierlicher oder periodischer Weise den Durchsatz der Charge in Verbindung mit der Messung der volumenbezogenen Masse des Feststoffs einstellt.

10. Verfahren nach einem der Ansprüche 1 bis 7, bei dem man in kontinuierlicher oder periodischer Weise die Menge des in die Reaktionszone eingeführten Feststoffs in Verbindung mit der Messung der volumenbezogenen Masse des Feststoffs einstellt.

11. Verfahren nach einem der Ansprüche 1 bis 7, bei dem man ein Schleppfluid in Strömungsrichtung vor dem Eintritt der Charge einführt und kontinuierlich oder periodisch ihren Durchsatz über Rechnermittel in Verbindung mit der volumenbezogenen Masse des Feststoffs einstellt.

12. Verfahren nach einem der Ansprüche 1 bis 7 und 11, bei dem man gleichzeitig kontinuierlich oder periodisch die Menge des in die Reaktionszone eingeführten Feststoffs sowie den Durchsatz des Mitreißefluids in Verbindung mit der Messung der volumenbezogenen Masse des Feststoffs und vorzugsweise mit der ihrer Geschwindigkeit, einstellt.

13. Verfahren nach einem der Ansprüche 1 bis 12, bei dem die Reaktionszone eine Vielzahl von Einführungspunkten der Charge umfaßt, über die man eine Chargenmenge in Bezug auf die volumenbezogene Masse des Feststoffs einführt, die benachbart diesem Einführungspunkt der Charge gemessen wurde und man diese Chargenmenge als Funktion der gemessenen volumenbezogenen Masse reguliert.

PL_unique

**FIG.1**

**FIG.2**

**FIG.3**